# DEMANDE DE BREVET EUROPEEN

(11) **EP 0 700 933 A1**
(43) Date de publication de la demande: **13.03.1996**
(21) Numéro de dépôt: 95402027.7
(22) Date de dépôt: 07.09.1995
(51) Int. Cl.: C08F 8/34, C08C 19/22, G01N 33/545

(54) **Microsphère de latex biotinylée, procédé de préparation d'une telle microsphère et utilisation en tant qu'agent détection biologique**

(30) Priorité: 09.09.1994 FR 9410789
(71) Demandeur: SOCIETE PROLABO, F-94120 Fontenay-sous-Bois (FR)
(72) Inventeur: Richard, Joel, 15 Avenue Marie-Amélie, 60500 Chantilly (FR); Vaslin, Sophie, F-94360 Bry-sur-Marne (FR)
(74) Mandataire: Warcoin, Jacques

(57) **Abrégé**

L'invention concerne une microsphère de latex constituée de polymères obtenus par polymérisation de monomères insaturés éthyléniquement, présentant des groupements fonctionnels en surface, remarquable en ce que sur au moins une partie desdits groupements sont greffés, par l'intermédiaire d'une chaîne divalente, des radicaux biotinyle.

Elle concerne également le complexe avidine- ou streptavidine-biotine obtenu à l'aide de ladite microsphère et l'utilisation de la microsphère ou du complexe en tant qu'agent de diagnostic de dosage biologique ou de dosage immunologique.

## Description

La présente invention concerne une nouvelle microsphère de latex biotinylée, un complexe avidine- ou streptavidine-microsphère biotinylée, un procédé de préparation d'une telle microsphère ainsi que l'application de la microsphère de latex biotinylée ou du complexe avec l'avidine ou la streptavidine comme agent de diagnostic ou de dosage biologique (immunologique, enzymatique, etc) ainsi qu'en biologie moléculaire.

L'interaction (strept)avidine-biotine est utilisée depuis de nombreuses années dans des applications biologiques variées, notamment dans le domaine de la séparation et du séquençage d'ADN (.....), de l'hybridation des acides nucléiques, des dosages immunologiques, du marquage cellulaire et du tri cellulaire.

Les avantages du système de reconnaissance streptavidine-biotine ou avidine biotine sont nombreux et bien connus ; il s'agit de :
- la création d'une interaction forte entre ces deux molécules, qui assure la stabilité du complexe dans des conditions variables de pH au cours des différentes étapes de lavage, couplage et analyse,
- la réduction du couplage non spécifique,
- la relative facilité d'introduction de la biotine dans les macromolécules biologiques sans modification de leur activité, en vue du couplage sur le support streptavidine.

La biotine, aussi appelée vitamine H, de formule : 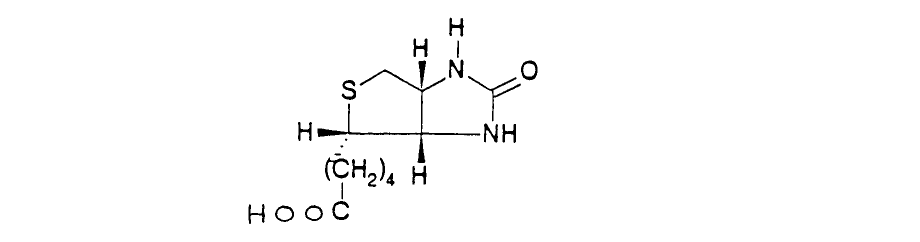
présente l'avantage de pouvoir se lier avec une sélectivité très élevée à l'avidine ou la streptavidine (kd = 1,3.10⁻¹⁵ mole/l).

L'avidine est une glycoprotéine présente dans le blanc d'oeul, possédant quatre sous-unités identiques de 128 aminoacides chacune et un point isoélectrique de 10. Elle peut fixer jusqu'à 4 molécules de biotine, soit une biotine par sous-unité. Par le terme "avidine", on souhaite également désigner la forme non glycosylée de cette protéine qui a essentiellement les mêmes caractéristiques d'affinité que la protéine glycosylée.

La streptavidine est une protéine provenant de la culture de Streptomyces avidinii possédant également quatre sous-unités identiques et un point isoélectrique d'environ 5-6.

Usuellement la streptavidine utilisée a subi une digestion par la protéinase K. Toutefois, dans le cadre de la présente description, on souhaite également désigner par l'expression "streptavidine" la forme native obtenue par exemple après clonage du gène de la streptavidine (Argarana CE, Nucleic Acids Res. 1986 ; 14:1871-1882).

On connaît par ailleurs l'application des microsphères de latex en tant que matériel de diagnostic médical. Par exemple, les particules de latex sont utilisées dans les tests d'agglutination et servent à détecter la présence ou l'absence d'anticorps ou d'antigène. Concrètement, un anticorps ou un antigène est lié à la surface d'une particule de latex qui peut par la suite réagir avec le ou les antigène(s) ou anticorps correspondant contenu dans un fluide corporel par exemple, le sérum du sang, le liquide céphalorachidien (LCR), l'urine, ou une préparation d'extrait tissulaire. Les méthodes de détection sont bien connues de l'homme du métier.

On a proposé dernièrement des latex contenant des microsphères magnétiques comportant des groupements streptavidine en surface et qui sont notamment utilisés dans le domaine du diagnostic médical et du dosage immunologique.

Néanmoins, un tel latex ne conduit pas à un résultat tout à fait satisfaisant, car la proximité de la surface des particules peut induire une dénaturation partielle de la streptavidine, qui diminue sa capacité de couplage avec la biotine, ou du moins rend celle-ci moins contrôlable.

L'invention propose un procédé de préparation de microsphères de latex biotinylées assurant :
a) un contrôle aisé du greffage covalent de la biotine modifiée, ce qui ouvre la possibilité d'obtenir des particules dont la concentration superficielle en biotine soit variée et connue,
   L'invention propose aussi de nouvelles microsphères :
b) offrant la possibilité de préparer indifféremment à partir d'une même base, des latex avidine ou des latex streptavidine : ce qui permet de proposer aux utilisateurs deux produits de valeur ajoutée différente. La streptavidine est plus coûteuse, mais elle donne moins d'interférences que l'avidine à cause de son point isoélectrique plus faible (avoisinant 5-6) et parce qu'elle est normalement dépourvue de résidus glycosylés qui sont présents à la surface de chaque sous-unités dans l'avidine.
c) permettant l'élimination des contraintes stériques dues à la proximité de la phase solide que représente le support polymère,
d) favorisant la formation du complexe biotine-streptavidine, ou biotine-avidine.
e) permettant de conserver la capacité de fixation de trois conjugués biotinylés.

L'invention concerne en premier lieu une microsphère de latex constituée de polymères obtenus par polymérisation de monomères insaturés éthyléniquement, présentant des groupements fonctionnels en surface, et caractérisée en ce que sur au moins une partie desdits groupements sont greffés, par l'intermédiaire d'une chaîne divalente, des restes biotinyle.

L'ensemble reste biotinylé-chaîne divalente est aussi appelé "chaîne biotinylée".

Les microsphères de latex sont classiquement constituées de polymères obtenus par polymérisation de monomères éthyléniquement insaturés et sont fonctionnalisées en surface.

Il s'agit d'homopolymères ou de copolymères contenant des motifs dérivés de monomères vinylaromatiques, éthyléniques, d'acides ou d'esters alcanoïques ou éthyléniques dont une proportion est fonctionnalisée.

Ce type de polymères est facilement accessible à tout homme de l'art et on se contentera d'en citer quelques uns ci-après, à titre non limitatif. Il peut s'agir de :
- monomères éthyléniques de type isoprène, 1,3 butadiène, chlorure de vinylidène, acrylonitrile,
- monomères vinylaromatiques comme le styrène, le bromostyrène, l'alphaméthylstyrène, l'éthylstyrène, le vinyltoluène, le chlorostyrène ou le chlorométhylstyrène ou le vinylnaphtalène,
- acides, esters ou anhydrides alcénoïques commes les acides acrylique, méthacrylique, acrylates et méthacrylates d'alkyle dont le groupe alkyle possède 3 à 10 atomes de carbone, hydroxyalkylacrylates, les acrylamides, les esters d'acides éthyléniques à 4 ou 5 atomes de carbone ainsi que,
- les monomères difonctionnels tels que le divinylbenzène ou le diacrylate de 2,2-diméthyl-1,3-propylène et/ou d'autres monomères copolymérisables insolubles dans l'eau.

Une partie des monomères porte des groupements susceptibles de réagir, directement ou indirectement, avec des groupements fonctionnels de type amine, ou carboxyle par exemple, portés par des molécules biologiques comme les protéines et les enzymes. A titre représentatif de ces groupes fonctionnels, on peut citer les halogènes, les groupements carboxyle, amine, isocyanate, aziridine, aldéhyde, sulfonyle et les fonctions époxy, chlorométhyle.

Les monomères, plus particulièrement utilisés dans le cadre de la présente invention appartiennent à la famille des arylènes et/ou des alkylènes. Il s'agit préférentiellement de composés vinylaromatiques tels que: styrène, alphaméthylstyrène, éthylstyrène, tertio-butylstyrène et vinyltoluène. De préférence, ces monomères sont substitués par un ou plusieurs groupements fonctionnels de type halogène, amine, alcoxy, carboxyle et/ou sulfonyle.

Ces monomères sont utilisés seuls ou en mélange entre eux en toute proportion.

Les particules de polymères peuvent être obtenues par la mise en oeuvre d'une quelconque technique de polymérisation comme la polymérisation en émulsion classique, en microémulsion, en suspension, en microsuspension, ou le cas échéant par polymérisation en milieu organique. Ces techniques familières à l'homme de l'art ne seront pas rappelées ici.

Les particules selon l'invention sont hydrophobes et possèdent de préférence une taille généralement comprise entre 0,01 et 20 microns et de préférence inférieure à 5 microns. Elles sont calibrées, monodisperses et présentes dans le latex à raison d'une quantité variant entre 0,05 à 30 % en poids du poids total du latex, préférentiellement entre 0,1 et 2 %.

Généralement, la densité surfacique de groupements fonctionnels par nanomètre carré de microsphère est compris entre environ 1 et environ 50.

Les microsphères de latex peuvent être magnétisables et dans ce cas, elles sont associées à des matériaux magnétisables comme ceci est par exemple décrit dans les brevets US 4 339 337, US 4 358 388, US 4 948 739.

Parmi les matériaux pouvant constituer les parties magnétisables des microsphères, on peut citer la magnétite, l'hématite, le dioxyde de chrome, les oxydes mixtes yttrium-fer, les ferrites telles que les ferrites de manganèse, nickel, manganèse-zinc ...., les alliages de cobalt, nickel, gadolinium, samarium-cobalt ... Les matériaux préférentiels sont généralement la magnétite et l'hématite.

La quantité de matériaux magnétisables contenus dans les microsphères correspond à environ 0,5 à 70 %, de préférence à environ 15 à 60 % du poids de la microsphère composite magnétisable.

Du fait de la présence de la chaîne divalente qui fonctionne comme un "bras espaceur" les contraintes stériques dues à la proximité de la phase solide que représente la microsphère sont éliminées. De plus, la protéine (avidine ou streptavidine), étant éloignée de la surface de la microsphère, conserve sa capacité de fixation de trois autres conjugués biotinylés.

De préférence, la chaîne divalente présente une longueur moyenne allant de 5 à 120 Å, avantageusement de 5 à 50 Å et plus avantageusement aux environs de 15 Å.

Par l'expression "reste biotinyle" on entendra un radical susceptible d'être conjugué avec l'avidine ou la streptavidine. Ce reste doit donc comporter la structure de formule : 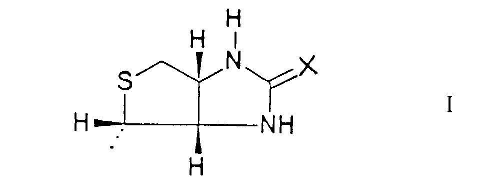
dans laquelle :
X est soit un atome d'oxygène, de soufre ou un radical imino N-H.

En général, ce reste sera constitué du radical biotinyle de formule : 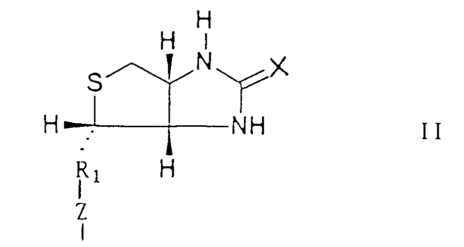
dans laquelle :
R₁ est un radical divalent hydrocarboné en C₁-C₈
X est soit un atome d'oxygène, de soufre ou un radical imino N-H.
Z est un groupe C = O ou -S-

De préférence, la chaîne divalente comporte de 3 à 80 atomes de carbone, avantageusement 8 à 20.

Il s'agit généralement d'un radical divalent hydrocarboné, éventuellement substitué, comportant éventuellement dans la chaîne principale un ou plusieurs hétéroatomes choisis parmi les atomes d'azote, d'oxygène, de soufre, de phosphore, un ou plusieurs des atomes de carbone pouvant être des groupes carbonyle ou dérivés (imine, oxime etc.) ou comportant un ou plusieurs cycles ou hétérocycles.

Les substituants du radical divalent sont ceux qui ne produisent pas d'empêchement stérique tel que le greffage sur les groupes fonctionnels de la microsphère deviendrait difficile voire impossible. Il s'agit donc de substituants de faible encombrement tel que les radicaux méthyle, amino, OH, etc.

De même, ces substituants ou les hétéroatomes faisant partie de la chaîne elle-même ne devront pas interférer dans la réaction entre le groupe terminal de cette chaîne et les groupes fonctionnels.

De façon générale, le nombre de chaînes biotinylées sur chaque microsphère de latex doit être suffisant pour permettre la mise en oeuvre d'un test de diagnostic.

Sur un plan pratique, ce nombre est une proportion plus ou moins importante de la quantité de groupes fonctionnels présents sur la microsphère.

Cette proportion varie avantageusement entre 1 et 50 % des groupes fonctionnels présents à la surface de la microsphère, de préférence la proportion est comprise entre 5 et 30 %.

Comme on l'a indiqué précédemment, la densité surfacique de groupements fonctionnels par nanomètre carré de microsphères est compris entre 1 et 50, ledit nombre est aisément déterminable.

Selon une première variante, la microsphère de latex présente la structure suivante :

M- [CO - NH - R - NH - B ]ₙ III

dans laquelle :
n représente le nombre moyen de molécules greffées par unité de surface,
M représente la microsphère de latex
B représente la chaîne biotinylée.
R est un radical divalent hydrocarboné, éventuellement substitué comportant éventuellement un ou plusieurs hétéroatomes choisis parmi les atomes d'azote, d'oxygène, de soufre, de phosphore, un ou plusieurs groupes carbonyle ou dérivés, un ou plusieurs cycles ou hétérocycles.

De préférence B correspond à la formule II ci-dessus où Z est C = O. De préférence encore, R₁ est le radical divalent (CH₂)₄.

Néanmoins, l'invention n'est pas limitée à cette forme particulière de reste biotinyle et peut également s'étendre aux restes dont la chaîne 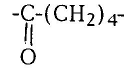
peut être remplacée par une chaîne équivalente.

De préférence, parmi les nombreux radicaux divalents R pouvant convenir dans la réalisation des microsphères selon l'invention, on peut citer le reste divalent de la cadavérine, l'éthylènediamine, l'hexaméthylènediamine ou un dihydrazide tel que l'adipodihydrazide, un reste de peptide, un reste d'acide aminé basique tel que la lysine, de préférence un reste de formule : 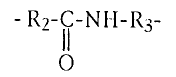
dans laquelle R₂, R₃ identiques ou différents sont une chaîne divalente en C₁-C₈, de préférence C₂-C₆, avantageusement C₅.

On a déjà indiqué que la densité surfacique des groupes fonctionnels sur la surface de la microsphère était avantageusement comprise entre 1 et 50 par nanomètre carré

De préférence, le pourcentage de bras espaceurs biotinylés greffés sur les groupes fonctionnels superficiels est compris entre 1 et 50 %. Avantageusement, le pourcentage est compris entre 5 et 30 %.

Le nombre n peut donc être déterminé compte tenu du diamètre de la sphère et est de façon générale compris entre environ 0,1 et environ 10.

Selon une seconde variante, la microsphère de latex présente la structure suivante :

M - [NH - CO - R-B]ₙ IV

dans laquelle M, n ont la même signification que dans la structure III. B représente la chaîne biotinylée,
R représente un radical divalent hydrocarboné, éventuellement substitué comportant éventuellement un ou plusieurs hétéroatomes choisis parmi les atomes d'azote, d'oxygène, de soufre, de phosphore, un ou plusieurs groupes carbonyle ou dérivés, un ou plusieurs cycles ou hétérocycles.

De préférence, B correspond à la formule II présentée ci-dessus où Z est S. De préférence encore R₁ est le radical divalent (CH₂)₄.

Néanmoins, l'invention n'est pas limitée à cette forme particulière de reste biotinyle et peut également s'étendre aux restes dont la chaîne -S-(CH₂)₄- peut être remplacée par une chaîne équivalente.

On a déjà indiqué que la densité surfacique des groupes fonctionnels sur la surface de la microsphère était avantageusement comprise entre 1 et 50 par nanomètre carré.

De préférence, le pourcentage de bras espaceurs biotinylés greffés sur les groupes fonctionnels superficiels est compris entre 1 et 50 %. Avantageusement, le pourcentage est compris entre 5 et 30 %.

Le nombre n peut donc être déterminé compte tenu du diamètre de la sphère et est de façon générale compris entre environ 0,1 et environ 10.

De préférence, parmi les nombreux radicaux divalents R pouvant convenir dans la réalisation des microsphères selon l'invention, on peut citer le reste divalent :

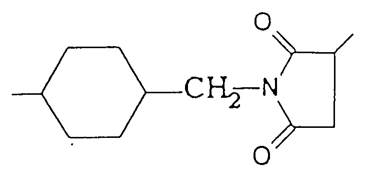

Bien entendu, d'autres groupes équivalents peuvent convenir.

L'invention concerne également les complexes microsphère biotinylée-avidine ou streptavidine caractérisés en ce que la microsphère est choisie parmi celles qui ont été décrites précédemment.

Elle concerne plus particulièrement une microsphère dans laquelle chaque reste biotinyle est conjugué à une avidine ou streptavidine.

L'invention concerne également un procédé de préparation de la microsphère ou du complexe tel qu'ils viennent d'être décrits précédemment caractérisé en ce qu'on effectue les étapes suivantes :
a) activation des groupements fonctionnels,
b) greffage de la chaîne biotinylée dont l'extrémité terminale est réactive vis-à-vis du groupement fonctionnel activé,
c) éventuellement complexation avec l'avidine ou la streptavidine.

De préférence, afin d'effectuer l'étape c) pour obtenir le complexe microsphère biotinylé-avidine ou streptavidine, on utilisera un excès molaire d'avidine ou de streptavidine par mole de biotine (avantageusement aux environs de 2).

Le couplage (complexation) est effectué de manière connue par exemple en présence d'un tampon phosphate + sérum albumine bovine suivi d'un lavage par un tampon phosphate.

On décrit maintenant deux modes de réalisation particuliers permettant la préparation respectivement des microsphères de structure III et IV.

Selon un procédé de préparation des microsphères biotinylées de structure III la première étape consiste à activer le latex carboxylique par synthèse d'un ester actif de sulfo N-hydroxysuccinimide (S-NHS) en présence de carbodiimide , notamment le CMC ou 1-cyclohexyl 3-(2-morpholino ethyl), carbodiimide meta-p-toluène sulfonate ou le EDC (1-éthyl-3-(3-diméthylamino-propyl carbodiimide méthiodide) selon la réaction suivante :

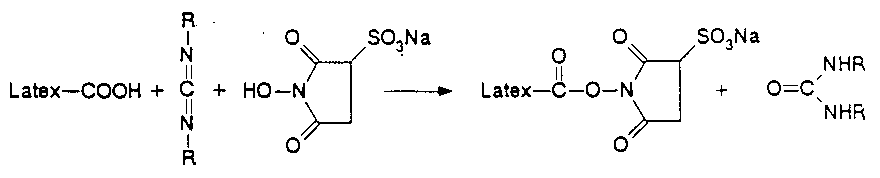

La réaction est effectuée en solution aqueuse. La réaction du carbodiimide sur les latex carboxyliques dans l'eau pure est quasi-instantanée à condition que le carbodiimide soit utilisé en excès.

Selon un mode opératoire préféré, on ajoute une solution aqueuse de S-NHS immédiatement après le carbodiimide. La réaction peut être contrôlée en suivant la disparition de S-NHS par spectrométrie d'absorption UV visible (λ max = 268 nm ; coefficient d'extinction molaire :
ε = 7500 mol⁻¹.1.cm⁻¹ en tampon phosphate pH = 7).

Pour cette étape de préparation de l'ester activé, il est préférable pour obtenir une réaction rapide et aussi complète que possible, de travailler avec un excès molaire d'EDC notamment compris entre 2 et 20, de préférence compris entre 4 et 10 (par rapport aux fonctions -COOH du latex) et un excès molaire de S-NHS notamment supérieure à la concentration en EDC, c'est-à-dire compris entre 5 et 100 (par rapport aux fonctions -COOH du latex) de préférence entre 7 et 50 pour une durée d'activation de 15 à 20 minutes. L'excès de S-NHS est éliminé par plusieurs lavages à l'eau ou avec une solution aqueuse de NaCl (0,25 M par exemple) jusqu'à ce que le surnageant ait une DO négligeable.

La deuxième étape consiste dans le greffage de la chaîne biotinylée à terminaison amine sur le latex activé par formation d'une liaison amide selon la réaction suivante :

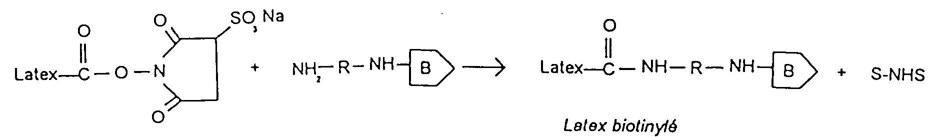

De préférence, la réaction est effectuée de manière à ce qu'il y ait une proportion sensiblement stoechiométrique de chaîne biotinylée par rapport au nombre de fonctions -COOH présentes sur la microsphère. Le greffage d'une amine sur le latex activé peut être contrôlé par dosage du S-NHS libéré lors de la réaction de transamidification. La réaction est effectuée en tampon à pH basique notamment aux environs de 9, notamment un tampon borax, mais peut aussi être effectuée dans l'eau ou dans une solution aqueuse de NaCl.

Le procédé de préparation des composés biotine-chaîne divalente est effectué par des voies de synthèse connues de l'homme du métier à partir de diamines commerciales dont un radical NH₂ est éventuellement masqué.

Un procédé de préparation d'une microsphère de structure IV est caractérisé en ce qu'il comprend :
a) l'activation du latex aminé par un bras espaceur hétérobifonctionnel de type sulfosuccinimidyl 4-(N-maléimidoalkyl) arylester tel que le sulfosuccinimidyl 4-(N-maléimidométhyl) cyclohexane-1 carboxylate ou S-SMCC, ou l'ester maleimido sulfosuccinimidyl de l'acide caproique,
b) le greffage sur le latex activé de la chaîne biotinylée à terminaison -SH.

L'activation du latex aminé est réalisée par exemple sous l'action du sulfosuccinimidyl 4-(N-maléimidométhyl) cyclohexane-1 carboxylate ou S-SMCC, de formule : 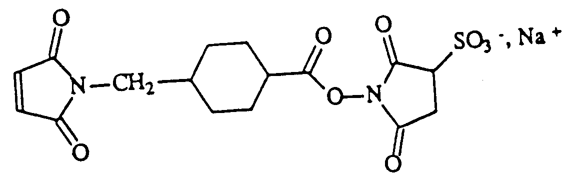
à pH neutre ou légèrement basique. La réaction entre le latex aminé et le S-SMCC est suivie par spectrophotométrie U.V. Sur des échantillons centrifugés 1 pic à 268 nm dû à l'apparition de S-NHS peut être mis en évidence.

La réaction avec le bras espaceur biotinylé à terminaison sulfhydryle consiste dans la réaction suivante :

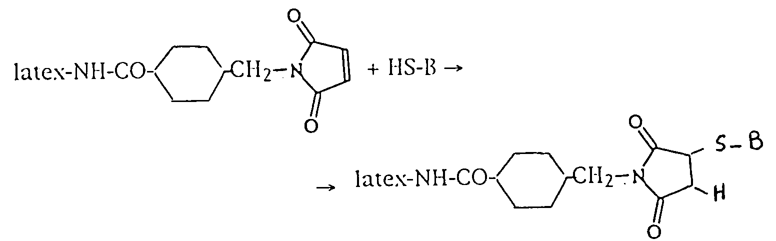

L'invention a enfin pour objet l'utilisation des microsphères ou des complexes précédemment décrits comme agent de diagnostic ou de détection.

Les systèmes avidine ou streptavidine-biotine peuvent être utilisés dans des domaines biologiques très variés. Les complexes ou microsphères décrits précédemment peuvent être notamment utiles dans les tests d'hybridation d'acides nucléiques, de dosage immunologique ou de dosage enzymatique.

Selon un premier aspect, les microsphères peuvent être utilisées dans les domaines de la séparation d'ADN.

Dans cette variante, la microsphère est mise en présence de l'avidine ou de la streptavidine et du brin d'ADN biotinylé. Cette méthode présente l'avantage de pouvoir mettre à profit les multiples sites de liaison de l'avidine ou la streptavidine avec la biotine.

La préparation de l'ADN biotinylé est effectuée de manière connue par amplification par la méthode PCR en faisant intervenir une proportion appropriée de nucléotides biotinylés.

Il est également possible d'appliquer la même méthode à la détection immunologique en faisant intervenir à la place de l'ADN biotinylé une protéine liée à un anticorps biotinylé spécifique de celle-ci.

Ce système est bien entendu applicable à d'autres molécules comme la lectine, la protéine A etc.

Selon un deuxième aspect, les complexes sont directement impliqués dans les réaction ci-dessus, l'avidine ou la streptavidine n'étant plus sous forme libre.

Selon un troisième aspect, les complexes sont utilisés en tant que support d'affinité avidine ou streptavidine-biotine.

Les complexes immobilisés peuvent être utilisés pour purifier, récupérer, caractériser les espèces biologiques citées dans la variante précédente.

Par exemple, une protéine présente dans les cellules peut réagir avec un anticorps biotinylé, celle-ci pouvant ensuite être récupérée après lyse des cellules sur la colonne d'affinité formée de complexe selon l'invention.

L'élution peut être assurée par dissociation de la protéine d'avec le complexe.

De même, une sonde d'ADN radioactive fixée sur un ADN préalablement amplifié par PCR et biotinylé peut être immobilisée sur un tel support constitué des complexes selon l'invention. Le comptage radioactif indique le nombre de molécules d'ADN à détecter.

D'autres applications comprennent notamment le séquençage d'ADN en phase solide en combinaison avec la méthode PCR, le tri-cellulaire en combinaison avec des anticorps spécifiques de la cellule considérée et biotinylés (cf. Clin. Chem. 37/5, 625-636 (1991) P. Diamandis et al).

Bien entendu, d'autres applications peuvent être envisagées par l'homme du métier sans sortir du cadre de la présente invention.

Les exemples ci-après illustrent la présente invention.

### Exemple 1 : Préparation de latex magnétique biotinylé

Le latex magnétique utilisé est celui commercialisé par la société PROLABO sous la référence ESTAPOR M1. 070/60 qui présente les caractéristiques suivantes :
- diamètre moyen d'une bille 0,8 µm
- masse volumique estimée : 1,94 g.ml⁻¹
- pourcentage massique de latex en suspension 10 %
- pourcentage massique de ferrite (pigment magnétique) 60 %
- concentration superficielle des fonctions -COOH 197 µeq.g⁻¹ de latex sec
- surface d'une bille 2 µm
- volume d'une bille O,27µm³
- nombre de billes/g de latex sec 2.10¹
- nombre de fonctions -COOH/bille 6.21.10⁶
- densité des fonctions -COOH 31-COOH/nm.

Ces latex sont polydisperses.

Le latex est lavé préalablement à l'activation.

Dans 1 ml d'eau bidistillée, on introduit sous agitation (Vortex 400 t/min) 24 mg de microbilles lavées, ce qui donne une concentration de 474 µM en fonction carboxylique. Un excès molaire de 5 en EDC (1-éthyl-3-(3-diméthyl-amino-propyl carbodiimide méthiodide) est ajouté et un excès molaire de 50 en S-NHS est ajouté 1 minute après l'EDC.

En quinze minutes, la quantité de S-NHS nécessaire à la formation d'ester actif est totalement consommée. L'excès de S-NHS est éliminé par plusieurs lavages, par une solution aqueuse de NaCl 0,25 M, jusqu'à ce que le surnageant ait une DO négligeable.

Le greffage de la biotine-X-cadavérine a été réalisé dans les conditions suivantes :
- milieu : eau distillée (4 ml),
- biotine-X-cadavérine en quantité stoechiométrique par rapport aux fonctions -COOH initiales,
- agitation vortex (400 tours/min).

Le produit obtenu présente les caractéristiques suivantes :

Le pourcentage de latex greffé par la biotine-X-cadavérine est d'environ 7 % (exprimé en microéquivalents de fonctions biotine par rapport aux fonctions carboxyliques initiales).

Une solution de streptavidine peroxydase dans de l'eau bidistillée à 0,01 % d'azide de sodium (concentration finale) est ajoutée au latex biotinylé à raison de 2 moles de streptavidine peroxydase par mole de biotine greffée sur latex, soit 1 mg de streptavidine couplé à la peroxydase pour 2,1 µg de biotine.

Le couplage biotine-streptavidine est effectué en tampon PBS + BSA (phosphate 0,1 M pH 7,2 dans NaCl 0,25 M contenant 1 g/l de BSA (sérum albumine bovine)), ensuite le latex streptavidine est lavé quatre fois par tampon phosphate 0,1 M dans NaCl 0,25 M et stocké dans 1 ml de ce tampon.

### Exemple 2

### Préparation de latex carboxylique non magnétique biotinylé

Le latex non magnétique utilisé est celui commercialisé par la société PROLABO sous la référence ESTAPOR K1. 030 et présente les caractéristiques suivantes :
- diamètre d'une bille 0,3 µm
- masse volumique estimée : 1 g.ml⁻¹
- pourcentage massique de latex en suspension 10 %
- concentration superficielle des fonctions -COOH 237 µeq.g⁻¹ de latex sec
- surface d'une bille 0,28 µm
- volume d'une bille O,0139µm³
- nombre de billes/g de latex sec 7,9-10¹³
- nombre de fonctions -COOH/bille 2.10⁶
- densité des fonctions -COOH 7,1-COOH/nm.

Ces latex sont monodisperses.

Le latex est lavé préalablement à l'activation.

La réaction d'activation est faite entièrement dans l'eau bidistillée avec une concentration de 62 µM en fonction carboxylique, un excès de 7 en EDC et un excès de 3 en S-NHS, ajouté 1 minute après EDC.

En trois minutes, la quantité de S-NHS nécessaire à la formation d'ester actif est totalement consommée (DO = 0,68).

Le greffage de la biotine-X-cadavérine a été réalisé dans les conditions suivantes :
- milieu : eau distillée,
- biotine-X-cadavérine en quantité stoechiométrique par rapport aux fonctions -COOH initiales,
- agitation vortex (400 tours/min).

Le pourcentage de latex greffé par la biotine-X-cadavérine est d'environ 7 % (exprimé en microéquivalents de fonctions biotine par rapport aux fonctions carboxyliques initiales).

Une solution de streptavidine peroxydase dans de l'eau bidistillée à 0,01 % d'azide de sodium (concentration finale) est ajoutée au latex biotinylé à raison de 2 moles de streptavidine peroxydase par mole de biotine greffé sur latex pour obtenir le complexe.

## Revendications

1. Microsphère de latex, constituée de polymères obtenus par polymérisation de monomères insaturés éthyléniquement, présentant des groupements fonctionnels en surface, caractérisée en ce que sur au moins une partie desdits groupements sont greffés, par l'intermédiaire d'une chaîne divalente, des restes biotinyle.

2. Microsphère selon la revendication 1, caractérisée en ce que la chaîne divalente a une longueur moyenne allant de 5 à 120 Å.

3. Microsphère de latex selon la revendication 1 ou 2, caractérisée en ce que la chaîne divalente comporte de 3 à 80 atomes.

4. Microsphère de latex selon l'une des revendications 1 à 3, caractérisée en ce que le reste biotinyle est de formule : 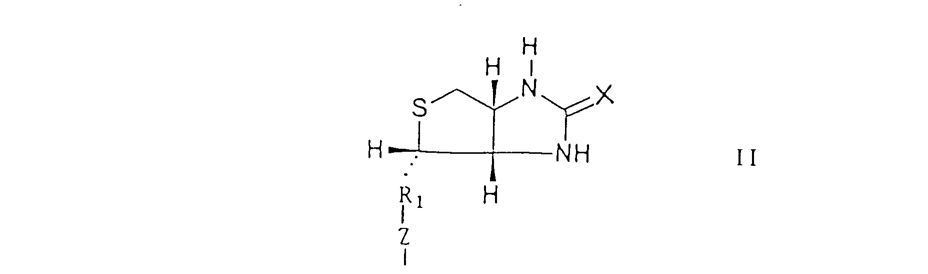 où
R₁ est un radical divalent hydrocarboné en C₁-C₈
X est soit un atome d'oxygène, de soufre ou un radical imino N-H.
Z est un groupe C = O ou -S-

5. Microsphère de latex selon l'une des revendications 1 à 4, caractérisée en ce qu'elle présente la structure suivante :
M - [ CO - NH - R - NH - B ]ₙ III
dans laquelle :
n représente le nombre moyen de molécules greffées par unité surfacique ;
notamment entre 0,1 et 10 par nanomètre carré,
M représente la microsphère de latex,
B est un reste biotinyle.
R est un radical divalent hydrocarboné, éventuellement substitué, comportant éventuellement un ou plusieurs hétéroatomes choisis parmi les atomes d'azote, d'oxygène, de soufre, de phosphore, un ou plusieurs groupes carbonyle ou dérivés, un ou plusieurs cycles ou hétérocycles.

6. Microsphère de latex selon la revendication 5, caractérisée en ce que le reste biotinyle est de formule :

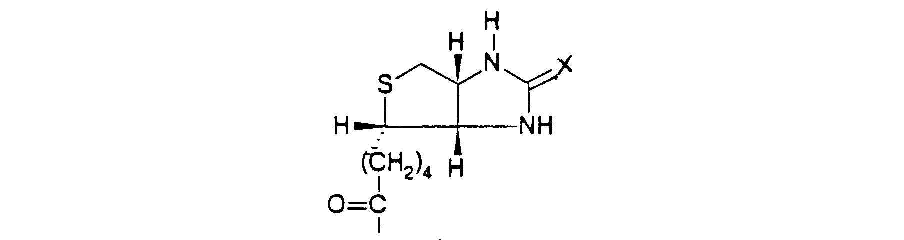

7. Microsphère de latex selon la revendication 6, caractérisée en ce que R est choisi dans le groupe constitué par le reste divalent de formule: 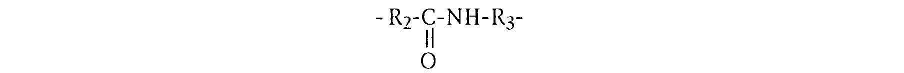 dans laquelle R₂, R₃ identiques ou différents sont une chaîne divalente en C₂-C₆.

8. Microsphère de latex selon l'une des revendications 1 à 4, caractérisée en ce qu'elle présente la structure suivante :
M - [ NH - CO - R - B ]ₙ IV
dans laquelle :
n représente le nombre moyen de molécules greffées par unité surfacique,
notamment entre 0,1 et 10 par nanomètre carré.
M représente la microsphère de latex,
B est un reste biotinyle.
R est un radical divalent hydrocarboné, éventuellement substitué comportant éventuellement un ou plusieurs hétéroatomes choisis parmi les atomes d'azote, d'oxygène, de soufre, de phosphore, un ou plusieurs groupes carbonyle ou dérivés, un ou plusieurs cycles ou hétérocycles.

9. Microsphère de latex selon la revendication 8, caractérisée en ce que le reste biotinyle est de formule :

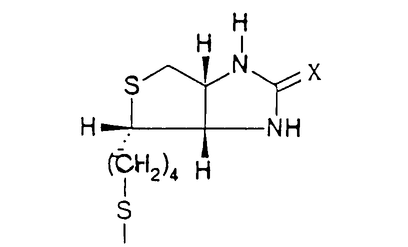

10. Microsphère de latex selon la revendication 8, caractérisée en ce que R est choisi dans le groupe constitué par le reste divalent de formule :

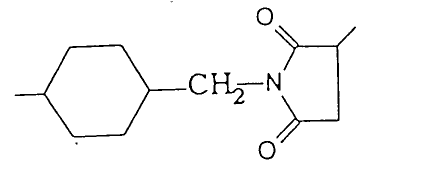

11. Microsphère de latex selon l'une des revendications précédentes, caractérisée en ce que la densité surfacique de groupements fonctionnels par nanomètre carré de microsphère est compris entre 1 et 50.

12. Microsphère selon la revendication 1 à 11, caractérisée en ce que le pourcentage de restes biotinyle par rapport au nombre de groupements fonctionnels présents sur la microsphère est compris entre 1 et 50%.

13. Complexe de microsphère de latex biotinylée et d'avidine ou de streptavidine, caractérisé en ce que la microsphère de latex biotinylée est conforme à l'une des revendications 1 à 12.

14. Complexe de microsphère de latex biotinylée et d'avidine ou de streptavidine selon la revendication 13, caractérisé en ce qu'environ une molécule d'avidine ou de streptavidine est fixée par reste biotinyle.

15. Procédé de préparation d'une microsphère selon l'une des revendications 1 à 12 ou d'un complexe selon l'une des revendications 13 ou 14, caractérisé en ce qu'on effectue les étapes suivantes :
a) activation des groupements fonctionnels de la microsphère,
b) greffage de la chaîne biotinylée dont l'extrémité terminale est réactive vis-à-vis du groupement fonctionnel activé,
c) éventuellement complexation avec l'avidine ou la streptavidine.

16. Procédé de préparation d'une microsphère de latex selon la revendication 15 d'une microsphère de structure III, caractérisé en ce qu'il comprend :
a) l'activation du latex carboxylique par le sulfo N-hydroxysuccinimide (S-NHS) en présence d'un carbodiimide.
b) le greffage sur le latex activé de la chaîne biotinylée à terminaison NH₂.

17. Procédé de préparation d'une microsphère de latex selon la revendication 15 d'une microsphère de structure IV, caractérisé en ce qu'il comprend :
a) l'activation du latex aminé par un bras espaceur hétérobifonctionnel de type sulfosuccinimidyl 4-(N-maléimidoalkyl) arylester,
b) le greffage sur le latex activé de la chaîne biotinylée à terminaison -SH.

18. Procédé de préparation selon la revendication 17, caractérisé en ce que le bras espaceur est le sulfosuccinimidyl 4-(N-maléimidoalkyl) cyclohexane-1 carboxylate ou S-SMCC.

19. Procédé de préparation selon l'une des revendications 15 à 18, caractérisé en ce que le complexe avec l'avidine ou la streptavidine est obtenu par mise en présence d'un excès molaire d'avidine ou de streptavidine.

20. Procédé de préparation selon la revendication 19, caractérisé en ce que l'excès molaire d'avidine ou de streptavidine est d'environ un facteur 2.

21. Utilisation des microsphères selon l'une des revendications 1 à 12 ou des complexes selon l'une des revendications 13 ou 14, comme agents de détection biologique.

22. Utilisation selon la revendication 21, caractérisé en ce que cette utilisation est destinée à l'hybridation, au séquençage d'ADN.

23. Utilisation selon la revendication 22, caractérisé en ce que cette utilisation est destinée à la purification de protéines ou au tri cellulaire.
